# EUROPEAN PATENT SPECIFICATION

(11) **EP 3 964 215 B1**
(45) Date of publication and mention of the grant of the patent: **25.11.2026**
(21) Application number: 20797986.5
(22) Date of filing: 29.04.2020
(51) Int. Cl.: A61K 31/655, A61K 9/00, A61P 17/00, A61P 17/06, A61P 29/00

(54) **PHARMACEUTICAL COMPOSITION, COMPRISING 6-DIAZO-5-OXO-L-NORLEUCINE, FOR TREATMENT OF INFLAMMATORY SKIN DISEASE**
PHARMAZEUTISCHE ZUSAMMENSETZUNG MIT 6-DIAZO-5-OXO-L-NORLEUCIN ZUR BEHANDLUNG ENTZÜNDLICHER HAUTERKRANKUNGEN
COMPOSITION PHARMACEUTIQUE, COMPRENANT DE LA 6-DIAZO-5-OXO-L-NORLEUCINE, POUR LE TRAITEMENT D'UNE MALADIE CUTANÉE INFLAMMATOIRE

(30) Priority: 30.04.2019 KR 20190050324
(43) Date of publication of application: 09.03.2022
(73) Proprietor: Dermato Bio Inc., Incheon 21984 (KR)
(72) Inventor: KIM, Min Jung, Seoul 03011 (KR); AHN, Jae Hyung, Seoul 08207 (KR)
(74) Representative: Eisenführ Speiser
(86) International application number: PCT/KR2020/005734
(87) International publication number: WO 2020/222552

(56) References cited:
- WO-A1-2014/160071
- CN-A- 112 807 297
- US-A1- 2016 051 674
- US-A1- 2016 051 674
- US-A1- 2018 221 337
- US-A1- 2018 221 337
- SIMPSON EL: "Atopic dermatitis: a review of topical treatment options", CURRENT MEDICAL RESEARCH AND OPINION, HANTS, GB, vol. 26, no. 3, 3 March 2010 (2010-03-03), pages 633 - 640, XP009140288, ISSN: 0300-7995
- OGAWA EISAKU ET AL: "Pathogenesis of psoriasis and development of treatment", JOURNAL OF DERMATOLOGY, vol. 45, no. 3, 10 December 2017 (2017-12-10), JP, pages 264 - 272, XP093218277, ISSN: 0385-2407, Retrieved from the Internet <URL:https://api.wiley.com/onlinelibrary/tdm/v1/articles/10.1111%2F1346-8138.14139> DOI: 10.1111/1346-8138.14139
- FARBER, E. M.: "Studies on the nature and management of psoriasis", CALIFORNIA MEDICINE, vol. 114, no. 6, 1971, pages 1 - 10, XP055756631
- VIGELAND, C. L. ET AL.: "Inhibition of glutamine metabolism accelerates resolution of acute lung injury. Physiological reports", vol. 7, no. 5, XP055756632
- MANIVANNAN, S. ET AL.: "Protective effects of glutamine antagonist 6-diazo-5-oxo-1-norleucine in mice with alphavirus encephalomyelitis", JOURNAL OF VIROLOGY, vol. 90, no. 20, 2016, pages 9251 - 9262, XP055756635

## Description

### [Technical Field]

The present invention relates to a pharmaceutical composition containing 6-diazo-5-oxo-L-norleucine or a pharmaceutically acceptable salt thereof for use in treating or preventing inflammatory skin diseases comprising atopic dermatitis and psoriasis.

### [Background Art]

Recently, inflammatory skin diseases that are emerging as a social problem include atopic dermatitis and psoriasis, and symptoms of these diseases may further worsen due to increased scratching caused by accompanying itching (pruritis).

To treat these skin diseases, treatment using steroids and calcineurin inhibitors, which mainly suppress the activity of immune cells, have been performed. For example, Korean Patent No. 10-0647857 discloses an external preparation for treating dermatitis, which includes a steroid, and International Patent Publication No. WO 2017-207819 discloses a composition for treating a skin disease, which includes a calcineurin inhibitor.

However, as a common dermatitis therapeutic agent, steroids (cortisol, prednisolone, methylprednisolone, dexamethasone injection and ointments and the like) have a side effect of a severe hypersensitivity reaction due to expanded capillaries and thinning of the skin layer, and also have a side effect of thinning and discoloration of the skin when a steroid-based anti-inflammatory drug is applied to a weak spot of the skin. Moreover, when steroid use is discontinued, a more severe atopic symptom called steroid rebound appears.

In addition, non-steroidal immunomodulators such as Elidel (pimecrolimus) cream and Protopic (tacrolimus, FK506) ointment have been developed, and when applied for a long period of time, have no side effects shown in conventional steroid ointments, and have been used for sensitive skin including the face and neck. However, recently, as concerns about the risk of cancer-causing calcineurin inhibitors have been raised, only low-dose use is permitted for patients under the age of 16, and even low-dose use is not permitted for children under the age of 2 or lower.

As other atopic therapies, an antihistamine agent has been used, and in severe cases, short-term administration of corticosteroids, the application of an external preparation, UV treatment on the skin or interferon treatment has been performed, but in most cases, inflammatory skin diseases are intractable diseases to the extent that they are only improved for a while and recur when drug use is discontinued.

In document US 2016/0051674 A1 methods and pharmaceutical compositions (ctps 1 inhibitors, e.g. norleucine) are described for inhibiting t cell proliferation in a subject in need thereof.

Document US 2018/0221337 A1 discloses methods and compositions for treating metabolic reprogramming disorders.

Document WO 2014/160071 A1 deals with methods and compositions for the treatment of glutamine-addicted cancers.

E.L. Simpson in Current Medical Research And Opinion, Hants, GB, vol. 26, no. 3, (March 2010) 633-640, discusses "Atopic dermatitis: a review of topical treatment options".

Ogawa Eisaku et al. in Journal of Dermatology, vol. 45, no. 3 (December 2017) 264-272, report of "Pathogenesis of psoriasis and development of treatment".

Therefore, there is an urgent need to develop a drug for treating atopic dermatitis and psoriasis, which has fewer side effects than conventional therapies and can be safely applied to children and infants, and at the same time, is economical.

### [Disclosure]

### [Technical Problem]

The present invention is directed to providing an economical and safe therapy, which has fewer side effects than steroids generally used to treat atopic dermatitis and psoriasis, can be used for a long time, and also is applied to infant and toddler patients. To this end, the present inventors attempted to discover a therapeutic agent for treating atopic dermatitis and psoriasis through a novel mechanism rather than the conventional mechanism of inhibiting immune cell activity or proliferation and test its efficacy.

### [Technical Solution]

To solve the above-described purpose, the present invention provides a pharmaceutical composition for use in treating or preventing an inflammatory skin disease, an autoimmune skin disease, an inflammatory sinus disease and/or an inflammatory lung disease, which includes 6-diazo-5-oxo-L-norleucine or a pharmaceutically acceptable salt thereof.

In one embodiment, the inflammatory skin disease may be an inflammatory skin disease caused by chemokine secretion from keratinocytes and fibroblasts or hyperproliferation of keratinocytes, and more specifically, an inflammatory atopic skin disease.

In one embodiment, the inflammatory skin disease may be accompanied by pruritus caused by mast cells.

In one embodiment, the inflammatory skin disease may be atopic dermatitis, eczema, contact dermatitis or psoriasis.

In one embodiment, the autoimmune skin disease may be lupus or Behcet's disease.

In one embodiment, the inflammatory sinus disease and/or inflammatory lung disease may be rhinitis, bronchitis, allergic asthma, a lung disease, a bacterial lung inflammatory disease, chronic obstructive pulmonary disease (COPD), pneumonia or lung cancer.

### [Advantageous Effects]

A pharmaceutical composition including 6-diazo-5-oxo-L-norleucine (DON) or a pharmaceutically acceptable salt thereof according to the present invention can effectively treat atopic dermatitis and psoriasis in the manner in which the 6-diazo-5-oxo-L-norleucine (i) inhibits chemokine secretion by inhibiting histone acetylation in keratinocytes and fibroblasts, (ii) inhibits hyperproliferation of keratinocytes and epithelial cells, (iii) inhibits mast cell infiltration and histamine secretion, and (iv) inhibits the penetration of immune cells into tissue by inducing the depletion of a metabolite of the extracellular matrix. The pharmaceutical composition works in a different way from the conventional therapeutic agent for a skin disease to be treated by inhibiting the activity and/or proliferation of immune cells.

In addition, due to considerably fewer side effects compared to conventional drugs, the pharmaceutical composition for use according to the present invention can be applied even to young child and infant-toddler patients, used for a long time, and can be effectively used for patients with severe symptoms and atopic march. In addition, the pharmaceutical composition for use according to the present invention can be applied by topical application with an ointment, administration with injection and a combination thereof, and can be more economically produced and used due to a low production cost.

### [Description of Drawings]

FIG. 1 is a graph showing the thickness of the epithelial layer of a mouse ear in a 12-O-tetradecanoylphorbol-13-acetate (TPA)-induced inflammation inhibiting experiment performed in one exemplary embodiment of the present invention.
FIG. 2 is a graph showing the thickness of the epithelial layer of an ear of an atopic dermatitis-induced mouse in an oxazolone-induced atopic dermatitis inhibiting experiment performed in one embodiment of the present invention.
FIG. 3 is a graph showing the NFκB activation capacity-inhibitory effect of DON induced by TNF-α in keratinocytes in an experiment of confirming chemokine inhibitory capacity performed in one embodiment of the present invention.
FIG. 4 is a graph showing the NFκB activation capacity-inhibitory effect of DON induced by TNF-α in fibroblasts in an experiment of confirming chemokine inhibitory capacity performed in one embodiment of the present invention.
FIG. 5 is a schematic diagram illustrating an experiment of inducing mouse inflammation using TPA according to one embodiment of the present invention.
FIG. 6 is a set of images showing skin conditions of mice after 5 days of the application of a therapeutic material in a TPA-induced inflammation inhibiting experiment according to one embodiment of the present invention.
FIG. 7 is a set of images showing the histological analysis result of a TPA-induced inflammation inhibiting experiment according to one embodiment of the present invention.
FIG. 8 is a graph obtained by scoring the area of an ear lesion visually evaluated in a TPA-induced inflammation inhibiting experiment according to one embodiment of the present invention.
FIG. 9 is a graph obtained by measuring the thickness and weight of ears of mice and a graph comparing the number of mast cells to confirm the antiinflammatory effect of DON in a TPA-induced inflammation inhibiting experiment according to one embodiment of the present invention.
FIG. 9C is a graph comparing the number of mast cells to confirm the antiinflammatory effect of DON in a TPA-induced inflammation inhibiting experiment according to one embodiment of the present invention.
FIG. 10 is a schematic diagram of an oxazolone-induced atopic dermatitis inhibiting experiment according to one embodiment of the present invention.
FIG. 11 is a set of images showing the ear skin of mice in which atopic dermatitis is induced in an oxazolone-induced atopic dermatitis inhibiting experiment according to one embodiment of the present invention.
FIG. 12 is a set of graphs obtained by measuring the weight and thickness of ear tissue of mice in which atopic dermatitis is induced in an oxazolone-induced atopic dermatitis inhibiting experiment according to one embodiment of the present invention.
FIG. 13 is a set of images showing the histological analysis result of an oxazolone-induced atopic dermatitis inhibiting experiment according to one embodiment of the present invention.
FIG. 14 is a set of graphs obtained by quantifying the total number of cells and the number of mast cells, which have infiltrated into the dermis, from the histological analysis result of an oxazolone-induced atopic dermatitis inhibiting experiment according to one embodiment of the present invention.
FIG. 15 is a graph obtained by measuring the thickness of ear tissue when ointment-type DON is applied in combination with dexamethazone in an oxazolone-induced atopic dermatitis inhibiting experiment according to one embodiment of the present invention.
FIG. 16 is a graph obtained by measuring the thickness of ear tissue when various concentrations of DON are subcutaneously injected in an oxazolone-induced atopic dermatitis inhibiting experiment according to one embodiment of the present invention.
FIG. 17 is a schematic diagram illustrating an imiquimod (IMQ)-induced psoriasis inhibiting experiment according to one embodiment of the present invention.
FIG. 18 is a set of images showing a back of mouse to which IMQ or IMQ+therapeutic material is applied in an IMQ-induced psoriasis inhibiting experiment according to one embodiment of the present invention.
FIG. 19 is a set of images showing the histological analysis result of an IMQ-induced psoriasis inhibiting experiment according to one embodiment of the present invention.
FIG. 20 is a graph obtained by scoring the keratin of a lesion visually evaluated in an IMQ-induced psoriasis inhibiting experiment according to one embodiment of the present invention.
FIG. 21 is a graph obtained by measuring the skin thickness in a lesion after an IMQ-induced psoriasis inhibiting experiment according to one embodiment of the present invention.

### [Modes of the Invention]

Hereinafter, embodiments of the present invention will be described in detail so that those of ordinary skill in the art can easily carry out the present invention. However, the present invention may be implemented in a variety of different forms, and is not limited to the embodiments described herein.

Throughout the specification, when one part "includes" a component, it means that it may also include other components rather than excluding components unless particularly stated otherwise.

In the specification, the term "approximately" or "substantially" used herein are used at, or in proximity to, numerical values when allowable manufacturing and material tolerances, which are inherent in the meanings, indicated are provided. These terms are used to prevent the unfair use of the disclosures in which correct or absolute values are cited to help in understanding the present invention by unscrupulous infringers. The term "step to" or "step of" used herein does not mean a "step for."

Throughout the specification, the term "combination thereof" included in the Markush-type expression refers to a mixture or combination of one or more selected from the group consisting of constituents described in the Markush-type expression, that is, one or more selected from the group consisting of the components.

The expression "A and/or B" used herein means "A, B or A and B".

Hereinafter, the pharmaceutical composition for use in treating or preventing an inflammatory skin disease, which includes 6-diazo-5-oxo-L-norleucine or a pharmaceutically acceptable salt thereof, according to the present invention will be described in detail with reference to embodiments, examples and drawings.

One aspect of the present invention relates to a pharmaceutical composition for use in treating or preventing an inflammatory skin disease, which includes 6-diazo-5-oxo-L-norleucine (DON) or a pharmaceutically acceptable salt thereof.

The pharmaceutical composition for use of the present invention may contain 6-diazo-5-oxo-L-norleucine or a pharmaceutically acceptable salt thereof as an active ingredient, and the term "active ingredient" used herein is a material exhibiting a physiological effect when a sufficient amount of the active ingredient is absorbed by the body of an organism.

According to one embodiment of the present invention, the inflammatory skin disease may be caused by chemokine secretion from keratinocytes and fibroblasts, but the present invention is not limited thereto. Specifically, the pharmaceutical composition for use of the present invention may inhibit chemokine secretion by inhibiting intracellular histone acetylation, and thus treat and/or prevent an inflammatory skin disease by inhibiting a TH2 reaction.

According to one embodiment of the present invention, the inflammatory skin disease may be caused by hyperproliferation of keratinocytes, but the present invention is not limited thereto. Specifically, the pharmaceutical composition for use of the present invention may prevent symptoms of the inflammatory skin disease due to keratinocyte hyperproliferation by inhibiting the granulation of keratinocytes.

According to one embodiment of the present invention, the inflammatory skin disease may be caused by infiltration of mast cells, but the present invention is not limited thereto. Specifically, the pharmaceutical composition for use of the present invention can inhibit histamine secretion from mast cells by inhibiting mast cell infiltration and histone acetylation, and thus treat and/or prevent an inflammatory skin disease, relieve a pruritus symptom, and prevent severe dermatitis due to secondary infection.

According to one embodiment of the present invention, the inflammatory skin disease may be caused by infiltration of immune cells, but the present invention is not limited thereto. Specifically, the pharmaceutical composition for use of the present invention may inhibit the infiltration of immune cells by inhibiting the expression and production of proteins essential for tissue penetration, such as an adhesion molecule or matrix metalloproteinase (MMP), and thus exhibit an effect of treating and/or preventing an inflammatory skin disease.

According to one embodiment of the present invention, the inflammatory skin disease may be atopic dermatitis, eczema, contact dermatitis or psoriasis.

According to another embodiment of the present invention, the autoimmune skin disease may be lupus or Behcet's disease.

According to still another embodiment of the present invention, the inflammatory sinus disease and/or inflammatory lung disease may be rhinitis, bronchitis, allergic asthma, a lung disease, a bacterial lung inflammatory disease, chronic obstructive pulmonary disease (COPD), pneumonia or lung cancer.

The pharmaceutical composition for use of the present invention may further include a pharmaceutically acceptable carrier and/or filler, which are conventionally used in the preparation of a pharmaceutical composition. The pharmaceutically acceptable carrier is well known in the art. That is, those of ordinary skill in the art may easily obtain an acceptable carrier for the use of the means and method of the present invention. The pharmaceutically acceptable carrier includes the following materials, but the present invention is not limited thereto: water, a salt solution, alcohol, arabic gum, vegetable oil, benzyl alcohol, polyethylene glycol, gelatin, carbohydrate, such as lactose, amylose or starch, magnesium stearate, talc, silicic acid, viscous paraffin, white paraffin, glycerol, alginate, hyaluronic acid, collagen, perfume oil, fatty acid monoglyceride and diglyceride, pentaerythritol fatty acid ester, hydroxy methylcellulose, and polyvinyl pyrrolidone. The carrier may also include any of the materials listed in the following documents [Documents: Remington: The Science and Practice of Pharmacy (Gennaro and Gennaro, Eds, 20th edition, Lippincott Williams & Wilkins, 2000); Theory and Practice of Industrial Pharmacy (Lachman et al, eds., 3rd edition, Lippincott Williams & Wilkins, 1986); Encyclopedia of Pharmaceutical Technology (Swarbrick and Boylan, eds., 2nd edition, Marcel Dekker, 2002)]. The filler may be selected from powdered cellulose, sorbitol, mannitol, various types of lactose and phosphate, but the present invention is not limited thereto.

**In** addition, the pharmaceutical composition may further include a polymer, a glidant and/or a lubricant as needed. For example, the polymer may be selected from the following materials, but the present invention is not limited thereto: a hydrophilic or hydrophobic polymer, such as a cellulose derivative (e.g., methyl cellulose, hydroxypropyl cellulose, hypromellose or methylcellulose); polyvinylpyrrolidone (e.g., povidone, crospovidone or copovidone); polymethacrylate (e.g., Eudragit RS, RL); a lipophilic ingredient (e.g., glyceryl monostearate or glyceryl behenate); and other materials, such as hydroxypropyl starch, polyethylene oxide or carrageenan. Most frequently, a hydrophilic swelling polymer, e.g., hypromellose, with a suitable viscosity of more than 5%, and more preferably, more than 8% may be used. The glidant may be selected from colloidal silicon dioxide, talc, magnesium stearate, calcium stearate, aluminum stearate, palmitic acid, stearic acid, stearol, cetanol and polyethylene glycol, but the present invention is not limited thereto. The lubricant may be selected from stearic acid, magnesium stearate, calcium stearate, aluminum stearate, sodium stearyl fumarate, talc, hydrogenated castor oil and polyethylene glycol, but the present invention is not limited thereto.

The active ingredient, 6-diazo-5-oxo-L-norleucine, of the pharmaceutical composition for use of the present invention may be formulated as a composition in the form of a neutralized pharmaceutically acceptable salt. The pharmaceutically acceptable salt may include an acid addition salt, which is formed with an inorganic acid, such as hydrochloric acid, sulfuric acid or phosphoric acid, or an organic acid such as acetic acid, oxalic acid, tartaric acid, mandelic acid or citric acid, but the present invention is not limited thereto. In addition, the salt may be derived from inorganic bases, such as sodium, potassium, ammonium, calcium and ferric hydroxide, and an organic base, isopropylamine, trimethylamine, 2-ethylamino ethanol, histidine and procaine.

The composition for use according to the present invention is administered in a pharmaceutically effective amount. In the present invention, the "pharmaceutically effective amount" used herein refers to an amount sufficient for treating a disease at a reasonable benefit/risk ratio applicable for medical treatment, and an effective dosage may be determined by parameters including the severity of a disease, the activity of a drug, a patient's age, weight, health condition, gender or sensitivity to a drug, the administration time, route and excretion rate of the pharmaceutical composition for use of the present invention, the duration of treatment and drugs simultaneously used, and other parameters well known in the medical field. The pharmaceutical composition for use of the present invention may be administered alone or in combination with a known therapeutic agent for an inflammatory skin disease. In consideration of all of the above-mentioned parameters, it is important to achieve the maximum effect with the minimum dose without a side effect.

The "combined administration" means that two or more administrations are performed simultaneously or sequentially. That is, two or more -administrations may be administered in combination such that each administration is administered separately, simultaneously or sequentially (e.g., each administration is separated by a period of time). The period of time may be very short (e.g., substantially immediately after first administration) or longer (e.g., 1-60 sec, 1-60 min, 1-24 hr, or any value or range of values therebetween).

The dosage of the pharmaceutical composition for use of the present invention may be determined by those of ordinary skill in the art in consideration of the purpose of use, the severity of a disease, a patient's age, weight, gender, and medical history, or the type of a material used as an active ingredient. For example, the pharmaceutical composition for use of the present invention may be administered at approximately 0.1 ng to approximately 100 mg/kg, and preferably, approximately 1 ng to approximately 10 mg/kg per adult, and administration may be performed once a day or divided into several doses, but the present invention is not particularly limited thereto. The above dosage does not limit the scope of the present invention in any way. The pharmaceutical composition may be administered for approximately 1 week or more, 2 weeks or more, or 3 weeks or more, and for example, approximately 3 weeks.

For example, the pharmaceutical composition may be used for oral administration, intraperitoneal administration, intravenous administration, intramuscular administration, subcutaneous administration, intradermal administration, intranasal administration, intrapulmonary administration, local administration, topical administration or intrarectal administration, but the present invention is not limited thereto.

In one embodiment of the present invention, the pharmaceutical composition may be used for oral administration, injection or topical administration to a subject, but the present invention is not limited thereto. For injection, the pharmaceutical composition may be administered subcutaneously or intradermally, and for topical administration, it may be applied topically in the form of an ointment, but the present invention is not limited thereto.

For example, the pharmaceutical composition may be administered at 0.01 mg to 10 mg/kg, 0.05 mg to 5 mg/kg, or 0.1 mg to 5 mg/kg for injection, and for topical administration, administered at 0.1 mg to 10 mg/kg, 0.2 mg to 6 mg/kg or 0.1 mg to 5 mg/kg, but the present invention is not limited thereto.

According to one embodiment of the present invention, the pharmaceutical composition may be used for combined administration of subcutaneous injection and topical administration, or combined administration of intradermal injection and topical administration, but the present invention is not limited thereto.

When the pharmaceutical composition is administered by injection, it may be prepared as an injectable composition, particularly, using a sterilized aqueous solution, a non-aqueous solvent, a suspension, an emulsion or a freeze-dried formulation, and as the non-aqueous solvent or suspending agent, propylene glycol, polyethylene glycol, vegetable oil such as olive oil, or an injectable ester such as ethyl oleate may be used, but the present invention is not limited thereto. Particularly, for injection, the pharmaceutical composition may be mixed with a stabilizer or buffer in water, thereby preparing a solution or suspension, and may also be prepared for unit administration in an ampoule or vial.

When the pharmaceutical composition for use of the present invention is intended for topical administration, it may be a topical composition. The topical composition includes a formulation suitable for topical use, that is, application, for skin. For example, the composition includes 6-diazo-5-oxo-L-norleucine or a salt thereof, and a pharmaceutically acceptable topical carrier. For example, the pharmaceutically acceptable topical carrier may be included at approximately 50 to 99.99% based on the weight of the composition (e.g., approximately 80 to 95% based on the weight of the composition), but the present invention is not limited thereto.

The topical composition may be prepared in various types of products as follows, but the present invention is not limited thereto: a lotion, a cream, a gel, a stick, a spray, an ointment, a cleansing liquid wash, a solid bar, a shampoo, a paste, a powder, mousses, wipes, patches, a wound dressing, adhesive bands, a hydrogel, films, and cosmetics such as concealers, foundations, mascaras, and lipsticks. Such products include an emulsion (e.g., a microemulsion and a nanoemulsion), a gel, a solid, a micelle, and a liposome.

For example, the topical composition may be formulated as a solution. The solution typically includes an aqueous solvent (e.g., approximately 50 to 99.99%, for example, approximately 90 to 99% by weight of a pharmaceutically acceptable aqueous solvent). For example, the topical composition may be formulated as a solution including an emollient. Such a composition may contain an emollient at, preferably, approximately 2 to 50%. The "emollient" may include a material used to not only prevent or relieve dryness, but also protect the skin, and various types of suitable emollients are known and may be used in the present invention. The document, [Reference: the International Cosmetic Ingredient Dictionary and Handbook, eds. Wenninger and McEwen, pp. 1656-61, 1626, and 1654-55 (The Cosmetic, Toiletry, and Fragrance Assoc, Washington, D.C., 7th Edition, 1997) (hereinafter "ICI Handbook")] contains various examples of suitable materials.

A type of product that can be formulated as a solution is an ointment. The ointment may include an animal or vegetable oil or a simple base of a semi-solid hydrocarbon. The ointment may include approximately 0.1 to 2% of a thickening agent in addition to approximately 2 to 10% of a skin emollient. Examples of thickening agents or viscosity increasing agents, which can be used in the present invention, are disclosed in the document [ICI Handbook pp. 1693-1697]. In addition, the topical composition may be formulated as an emulsion. When a carrier is an emulsion, approximately 1 to 10% (e.g., approximately 2 to 5%) of the carrier may include an emulsifier, and the emulsifier may be a non-ionic, anionic or cationic emulsifier, and a suitable emulsifier is disclosed in the document [ICI Handbook, pp. 1673-1686].

The topical composition may also be formulated as a gel (e.g., aqueous gel) using a suitable gelling agent. The gelling agent suitable for an aqueous gel includes natural gum, acrylic acid and acrylate polymers and copolymers, and cellulose derivatives (e.g., hydroxymethyl cellulose and hydroxypropyl cellulose), but the present invention is not limited thereto. Gelling agents suitable for oils (e.g., mineral oil) include hydrogenated butylene/ethylene/styrene copolymers and hydrogenated ethylene/propylene/styrene copolymers, but the present invention is not limited thereto. Such a gel may typically contain such a gelling agent at approximately 0.1 to 5% based on the total weight of the gel, but the present invention is not limited thereto.

When the pharmaceutical composition is a topical composition, in addition to the above-described ingredients, very various oil-soluble materials and/or watersoluble materials, which are conventionally used for the skin, may be contained without particular limitation.

According to one embodiment of the present invention, the pharmaceutical composition may be injected into a subject at a dose of 0.1 to 5 mg/kg, but the present invention is not limited thereto. For example, the injection administration may be subcutaneous injection or intradermal injection. In this case, the pharmaceutical composition may be administered into a subject twice a week at a dose of 0.1 to 5 mg/kg, but the present invention is not limited thereto.

According to one embodiment of the present invention, the pharmaceutical composition may be used for topical administration to a subject at a dose of 0.1 to 5 mg/kg, and in this case, may be administered to a subject daily for 3 weeks at a dose of 0.1 to 5 mg/kg, and the dose may be applied once a day, or in divided doses two or three or more times a day, but the present invention is not limited thereto.

According to one embodiment of the present invention, the subject to which the pharmaceutical composition is applied may be a mammal such as a human, and preferably, a human.

A method of treating and/or preventing an inflammatory skin disease of a patient, which includes administering a pharmaceutical composition, which includes 6-diazo-5-oxo-L-norleucine or a pharmaceutically acceptable salt thereof, to a patient is disclosed but not claimed.

The patient may be an infant-toddler or young child at the age of 2 or less, but this method can also be applied to adolescent and adult patients, who are older than the infant-toddler, with atopic march.

Hereinafter, the present invention will be explained in further detail with reference to examples, but the following examples are merely provided to more easily understand the present invention, and not to limit the present invention.

### [Examples]

A lot of energy is consumed because macromolecules such as a protein, a lipid, and a nucleic acid must be produced during excessive cell proliferation and chemokine secretion. As essential energy sources for mammalian cells, carbon molecules such as glucose and glutamine are representative. From this, considering that glutamine is essential for an inflammatory disease, the inventors tried to prove that atopic dermatitis in an animal may be inhibited by suppressing glutamine.

DON is a glutamine antagonist, and interferes with glutamine metabolism. Due to the interference in glutamine metabolism by DON, the following experiment was performed to confirm that a dermatitis treatment effect can be exhibited without the suppression of immune cells.

### 1. Capacity of inhibiting hyperproliferation of epithelial cells

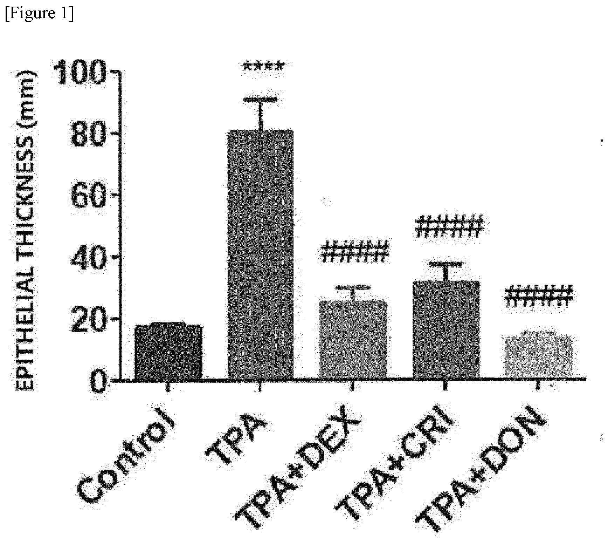

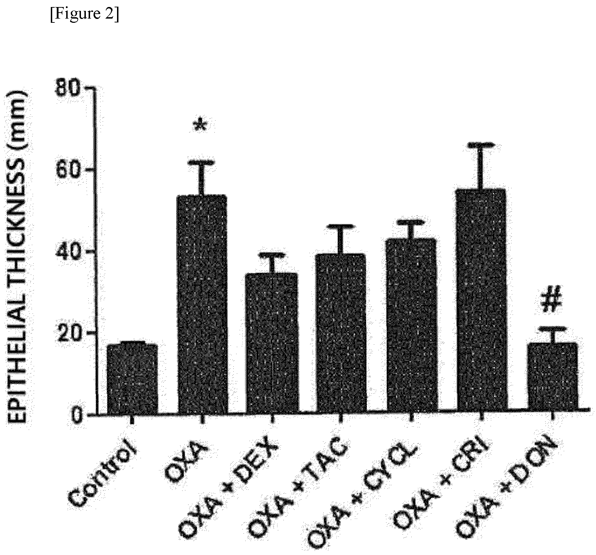

As shown in FIGS. 1 and 2, it was confirmed that the thickness of the epithelium was significantly reduced when an inflammatory lesion was treated with DON in a mouse in which inflammation was induced with TPA and oxazolone. From the result, it was expected that an effect of treating dermatitis such as atopic dermatitis and psoriasis may be exhibited by inhibiting abnormal growth of epithelial cells with DON.

### 2. Capacity of inhibiting chemokine in epithelial cells

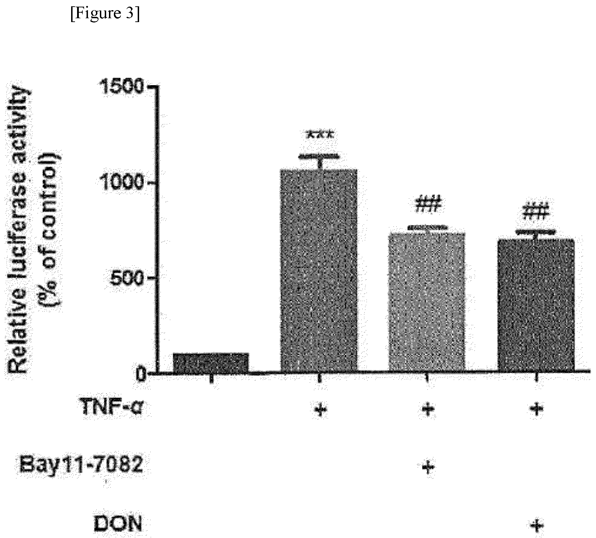

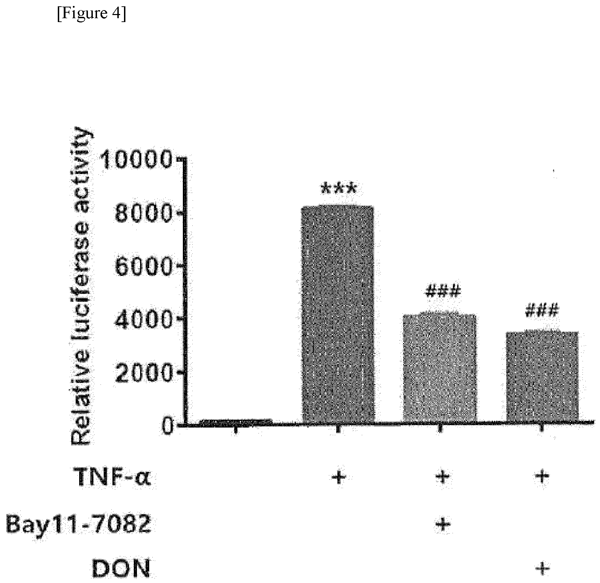

The capacity of inhibiting chemokines in epithelial cells was confirmed by measurement of NFκB activation capacity. NFκB is a transcription regulatory factor which regulates chemokine expression, and since it is a representative inflammation regulatory factor activated by various inflammation signals, it is possible to expect chemokine expression by confirming an NFκB signal. In FIGS. 3 and 4, it was confirmed that NFκB activity by TNF-α is inhibited by DON in each of keratinocytes and fibroblasts, and the inhibition of a Th2-type inflammatory chemokine expressed by NFκB activity was expected. From this result, glutamine metabolism in epithelial cells plays a very important role in the abnormal expression of inflammatory chemokines, and it was confirmed that the expression of inflammatory chemokines was inhibited by DON-induced inhibition of glutamine metabolism, thereby exhibiting an effect of treating an inflammatory skin disease.

### 3. Capacity of inhibiting immune cell penetration and capacity of inhibiting histamine secretion of mast cell

Based on the result that the number of immune cells penetrating into an atopic dermatitis-induced animal model was small in an experimental group treated with DON, the inventors assumed that DON would weaken the penetration force of immune cells, and tried to prove it. Generally, a lot of energy is required for penetration of immune cells into a lesion, this energy is maintained and replenished by absorbing amino acids or polysaccharides in the extracellular matrix (ECM). When immune cells to be infiltrated into a lesion absorb DON, glutamine metabolism is inhibited, and it was determined that there will be a problem in production of proteins essential for tissue penetration, such as an adhesion molecule or matrix metalloproteinase (MMP).

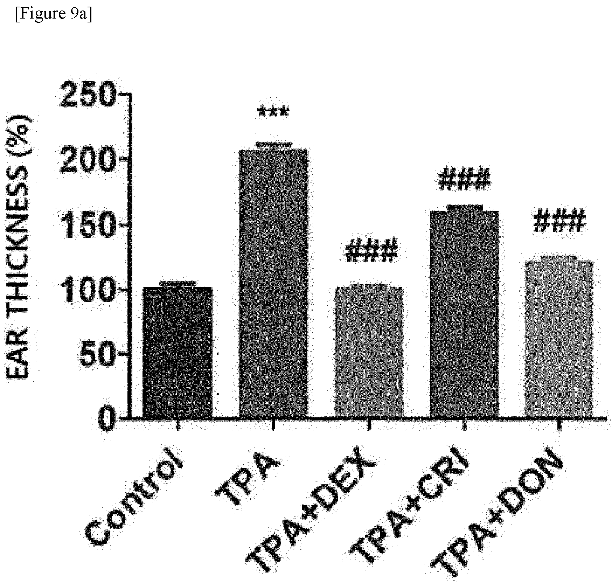

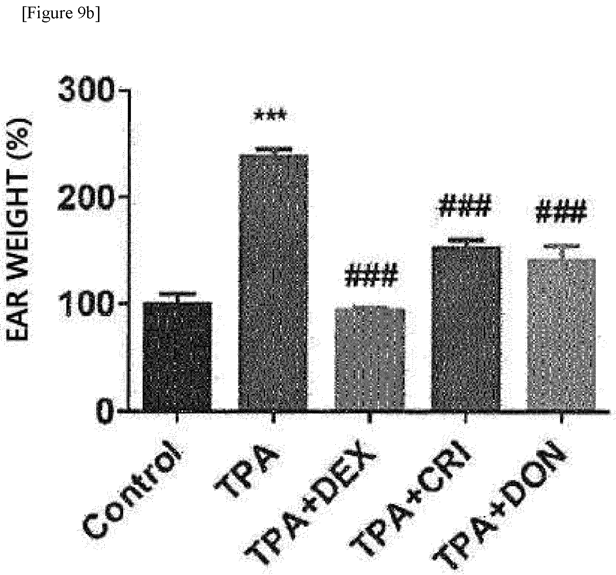

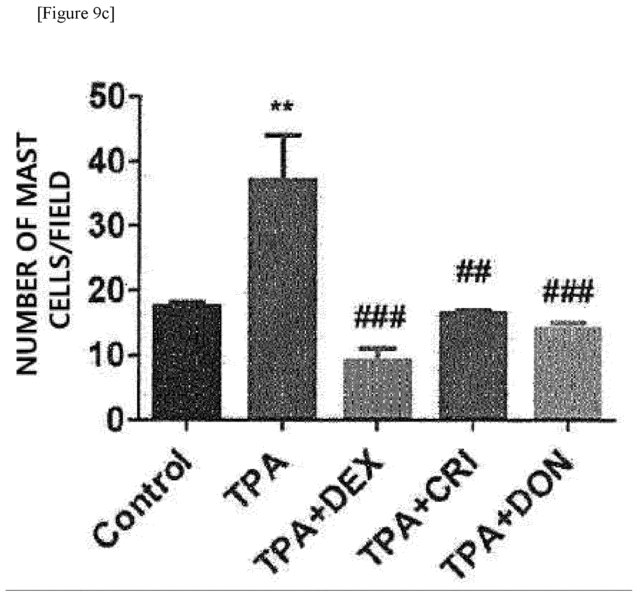

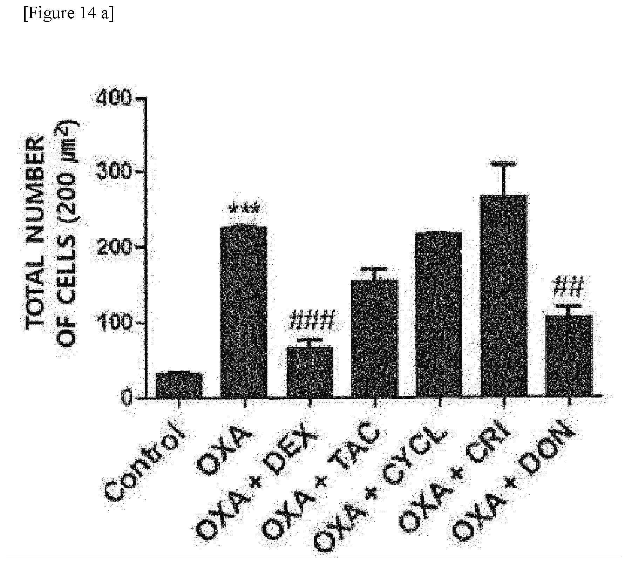

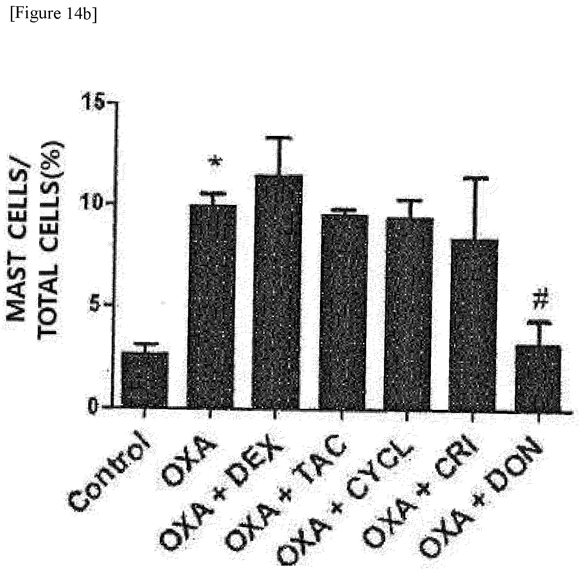

Mast cells are a type of immune cell. The infiltration of mast cells is frequently observed in an atopic lesion, and the mast cells secrete histamine to cause pruritis, which may become the cause of secondary infection or atopic march. Although many antihistamines have been developed to improve atopic symptoms, drugs that can directly control mast cells have not yet been developed. The mast cells require glycolysis activated by glutamine and glucose for activation. In DON treatment, as shown in FIGS. 9 and 14, it can be observed that the penetration of mask cells into a lesion of the dermatitis model is weakened. From this result, it was confirmed that DON inhibits not only epithelial cells but also mast cell infiltration, thereby relieving a pruritus symptom, and preventing severe dermatitis due to secondary infection.

### 4. Experiment of inhibiting TPA-induced inflammation

For an experiment of inducing and inhibiting inflammation using 12-O-tetradecanoylphorbol-13-acetate (TPA, Sigma), 14week-old C57BL/6 female mice were purchased from NARA Biotech (Korea) and used. The purchased mice were acclimated for 7 days, and healthy animals were selected and tested. During the experimental period, the mice were raised while maintaining the environment at a temperature of 21 to 25 °C, a relative humidity of 45 to 65% and a lighting time of 12 hours, and freely fed feed and drinking water.

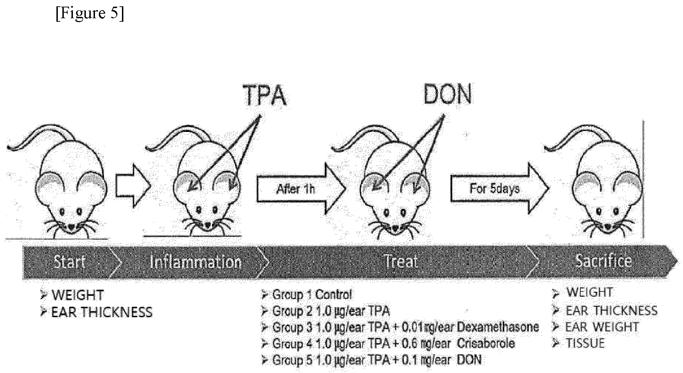

The schematic diagram of the experiment of inducing inflammation in a mouse using TPA is shown in FIG. 5. First, the acclimated mice with similar characteristics such as a body weight and size were divided into five experimental groups of 3, and separately raised to confirm an antiinflammation level. Inflammation was induced by first applying 1.0 µg of TPA to each of both ears of a mouse, and after 1 hour, a therapeutic material was applied, which was continued for 5 days. A mouse to which only acetone (Merck), which is a TPA solvent, was applied was set as a negative control (Vehicle), a mouse to which 1.0 µg of TPA dissolved in acetone was applied was set as a positive control and evaluated, and after TPA was applied, mice to which each of 3 mg of dexamethasone (DEX, Sigma), 0.6 mg of crisaborole (CRI, Chemscene) and 0.1 mg of 6-diazo-5-oxo-L-norleucine (DON, Sigma) was applied were set as experimental groups. After applying each material for 5 days by the above-described method, the weight, ear thickness and ear weight of each mouse were measured, and ear tissue was collected and histologically analyzed by hematoxylin & eosin (H&E) staining and toluidine blue staining.

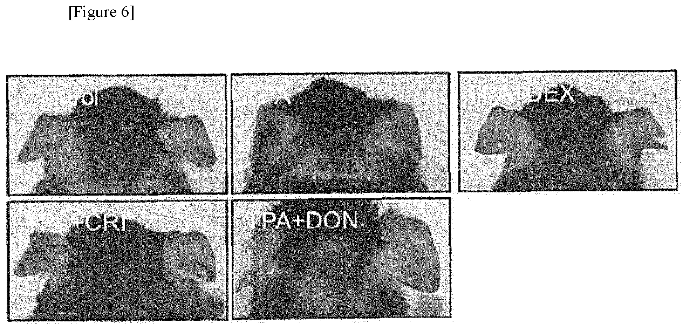

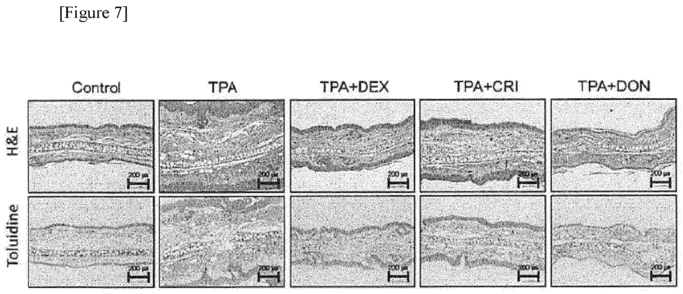

FIG. 6 is a set of images showing skin conditions of mice after 5 days of the application of a therapeutic material, and FIG. 7 is a set of images showing the histological analysis result of mice in the control and the experimental groups. Referring to FIG. 6, after TPA application, when experimental mice groups in which each of dexamethasone (Dexamethasone, DEX) crisaborole (Crisaborole, CRI) and DON was applied for 5 days, it was visually confirmed that the ear area showed erythema and scaling compared to a negative control. Referring to FIG. 7, in histological analysis, it was confirmed that the thickened skin tissue of the epidermis and dermis by TPA treatment becomes thinner due to DON.

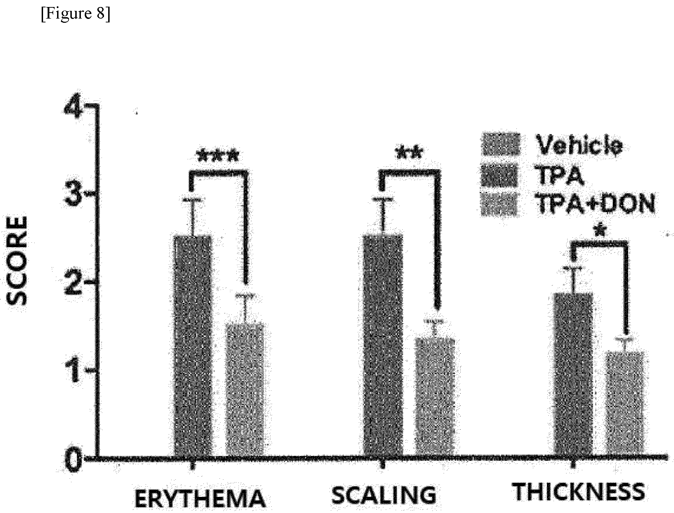

FIG. 8 is a graph obtained by scoring visually evaluated ear lesions, divided into erythema, scaling and a thickness, and in the graph, the severity of symptoms was rated on a 5-point scale (0 = none; 1 = slight; 2 = moderate; 3 = marked; and 4 = very marked). Referring to FIG. 8, it is confirmed that the degrees of erythema, scaling and thicknesses of the experimental group treated with DON were significantly reduced compared to a negative control.

Afterward, to confirm the antiinflammatory effect of DON, the mouse's ear thickness and ear weight were measured and quantified (FIGS. 9A and 9B), and, based on the histological analysis result obtained by toluidine blue staining capable of confirming mast cells, the number of mast cells was measured by comparing three tissue samples per group using a 200x image (FIG. 9C). As a result, it was confirmed that the number of mast cells was significantly increased in a TPA-treated positive control, compared with a negative control, and it was confirmed that the number of mast cells was significantly decreased in the dexamethasone (DEX) group, the crisaborole (CRI) group and the DON group, compared with the positive control. Therefore, it was confirmed that DON can alleviate pruritus symptoms by inhibiting the histamine secretion not only from epithelial cells but also from mast cells, and can also prevent severe dermatitis caused by secondary infection.

### 5. Experiment of inhibiting oxazolone-induced atopic dermatitis

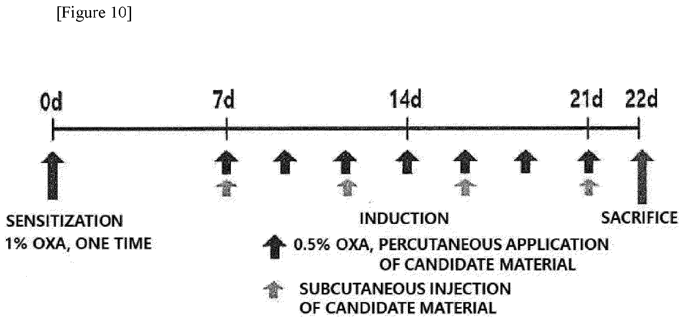

Using five 8-week-old female BALB/c mice per group, an effect of inhibiting oxazolone-induced atopic dermatitis was tested. In the experiment, dermatitis induced on the ear of each mouse was treated with a therapeutic candidate, and it was attempted to confirm an effect depending on an administration route of a therapeutic agent through percutaneous application or subcutaneous injection. First, to induce atopic dermatitis in all groups excluding the negative control, 0.35 mg of 4-ethoxymethylene-2-phenyl-2-oxazolin-5-one (oxazolone, OXA) was mixed with 30 µl of acetone, and sensitization was preceded on each of the both ears with the 1% mixture. The same amount of a solvent without OXA was applied for the negative control (vehicle). From 7 days after the initial application of OXA, OXA (0.5%) was additionally applied every two days to induce dermatitis. For treatment groups, dexamethasone (DEX), tacrolimus (TAC), cyclosporine (CYCL), crisaborole (CRI) and DON were used. For percutaneous application, each time OXA was applied, after 1 hour, each of the therapeutic candidates was treated on both ears, and in the administration test via subcutaneous injection, injection was performed 1 hour after OXA treatment, but intermittently performed every two days. As an excipient used in the subcutaneous injection, filtered distilled water was used, and the control was also administered the same amount of sterile water. Finally, on day 22 after the initial OXA application, the experiment was terminated, and the mice were sacrificed and autopsied, ear tissue was collected, and to confirm the morphological structure of the tissue, H&E staining was performed, and to count the number of the infiltrated mast cells, toluidine blue staining was performed. A schematic diagram of the experiment performed in this example is shown in FIG. 10.

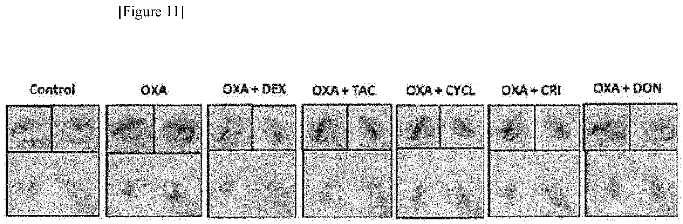

First, the experimental results for the mice treated according to the conditions listed in Table 1 below are shown in FIGS. 11 to 14.

**[Table 1]**

| **Group** | **Dose** | **Adminitration volume** | **Administration site and Method** |
|---|---|---|---|
| Control | - | 30 ul | ear, percutaneous application |
| OXA 0.5% | - | 30 ul | ear, percutaneous application |
| DEX 0.1% | 0.0272 mg | 30 ul | ear, percutaneous application |
| TAC 0.1% | 0.0272 mg | 30 ul | ear, percutaneous application |
| CYCL 0.1% | 0.0272 mg | 30 ul | ear, percutaneous application |
| CRI 0.1% | 0.0272 mg | 30 ul | ear, percutaneous application |
| DON 0.1% | 0.0272 mg | 30 ul | ear, percutaneous application |

FIG. 11 is a set of images of ears of mice in which atopic dermatitis is induced. Typical symptoms of atopic dermatitis accompanied by tissue swelling and eczema due to OXA treatment were observed, but it was confirmed that such symptoms were attenuated by treatment with a therapeutic material. Particularly, unlike the control OXA group, in the DEX group, the deformation of the ear tissue was most effectively suppressed, and significant skin improvement was also observed in the DON group.

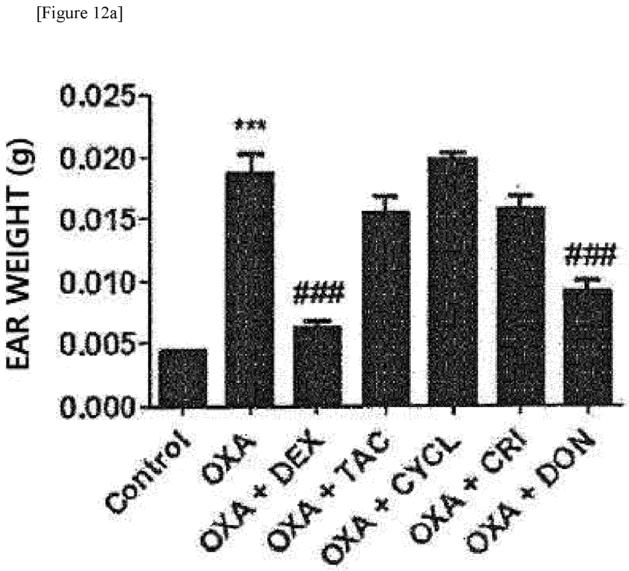

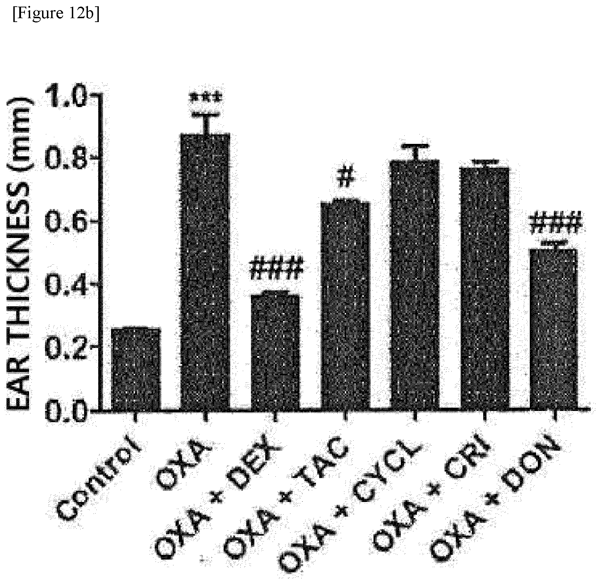

FIG 12 is a set of graphs showing the result of measuring a weight (g) and a thickness (mm) of a mouse in which atopic dermatitis was induced. Likewise, in the treatment groups such as a DEX group and a DON group, significantly decreased results are shown.

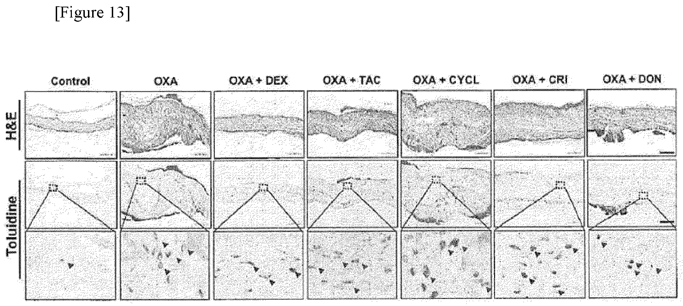

FIG. 13 is a set of images showing the histological analysis result of mouse ears in which atopic dermatitis is induced (X100, scale bar: 200 µm). From the H&E staining results, the morphological changes in skin were able to be observed, and from the toluidine blue staining results, the degree of mast cell infiltration was able to be confirmed. The mast cells were indicated by red arrows.

FIG. 14 is a set of graphs obtained by quantifying the total number of cells and the number of mast cells, which are infiltrated into the dermis, from the staining results. (a) The cell numbers were compared using the total number of infiltrated cells in 200 square micrometers (µm²), and (b) the mast cells infiltrated into the same area were converted into percentage (%) with respect to the total cell number.

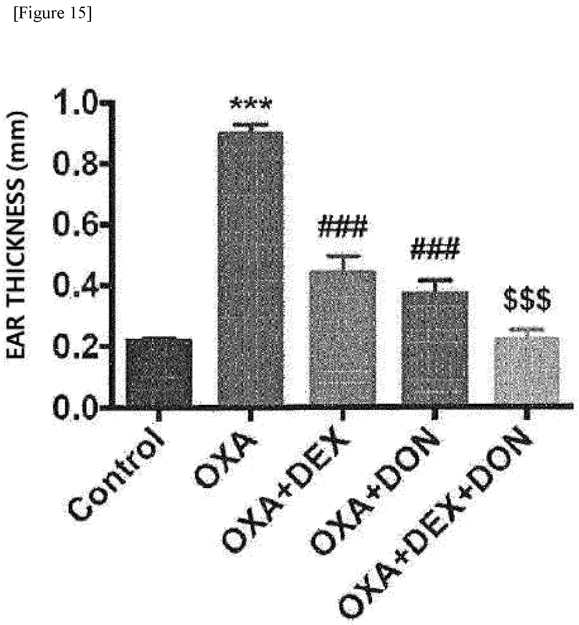

Next, the result of measuring the thickness of ear tissue of a mouse treated with a combination of the same concentrations of DEX and DON according to the conditions shown in Table 2 below is shown in FIG. 15.

**[Table 2]**

| **Group** | **Dose** | **Adminitration volume** | **Administration site and Method** |
|---|---|---|---|
| Control | - | 30 ul | ear, percutaneous application |
| OXA 0.5% | - | 30 ul | ear, percutaneous application |
| DEX 0.05% | 0.0136 mg | 30 ul | ear, percutaneous application |
| DON 0.05% | 0.0136 mg | 30 ul | ear, percutaneous application |
| DEX 0.05%+ DON 0.05% | 0.0136 mg (each) | 30 ul | ear, percutaneous application |

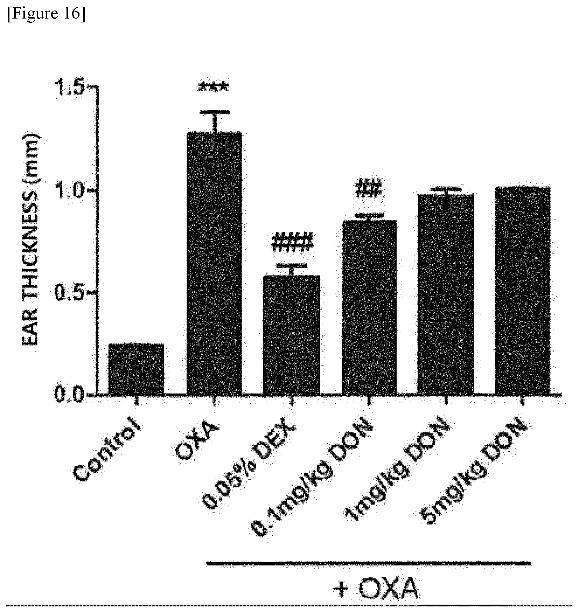

Afterward, the result of measuring the thickness of ear tissue of a mouse treated with DON at various concentrations through subcutaneous injection into the nape of the neck according to the conditions shown in Table 3 below is shown in FIG. 16.

**[Table 3]**

| **Group** | **Dose** | **Adminitration volume** | **Administration site and Method** |
|---|---|---|---|
| Control | - | 100 ul | nape of neck, subcutaneous injection |
| OXA 0.5% | - | 100 ul | nape of neck, subcutaneous injection |
| DEX 0.05% | 0.0136 mg | 100 ul | ear, percutaneous application |
| DON 0.01 mg/kg | 0.002 mg | 100 ul | nape of neck, subcutaneous injection |
| DON 1 mg/kg | 0.02 mg | 100 ul | nape of neck, subcutaneous injection |
| DON 5 mg/kg | 0.1 mg | 100 ul | nape of neck, subcutaneous injection |

According to this example, from the visual and histological analysis results, it was confirmed that DON significantly reduces changes in the epithelium of skin, the number of immune cells introduced into the dermis, and particularly the number of mast cells. Accordingly, DON may inhibit histological changes in epithelium and immunological inflammatory responses in the pathogenesis of atopic dermatitis, demonstrating that DON has potential as a therapeutic agent for atopic dermatitis.

### 6. Experiment of inhibiting IMQ-induced psoriasis

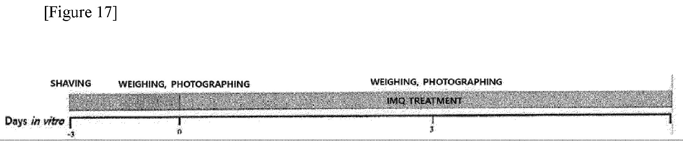

For a psoriasis inhibition experiment using imiquimod (IMQ, Tokyo Chemical Industry, I0747-100 mg), as an experimental animal, female C57/BL6 mice (n=5) were used. First, to induce psoriasis, sensitization was preceded by evenly applying 4 mg IMQ to the completely shaved back of each of the mice in all groups except a negative control at a volume of 100 µl. For the negative control, the same amount of a solvent without IMQ was applied. Psoriasis was induced by applying IMQ a total of five times at 24-hour intervals for 5 days, and for treatment groups, the same site was treated (applied) with 0.05% dexamethasone (DEX) (5 mg/ml, 100 µl) or 0.05% DON (0.5 mg/ml, 100 µl) dissolved in ethanol one hour after IMQ treatment. The schematic diagram of the experiment is shown in FIG. 17.

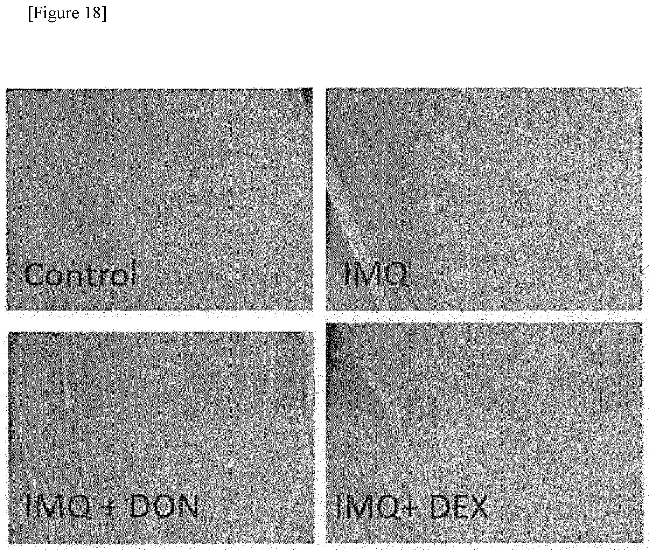

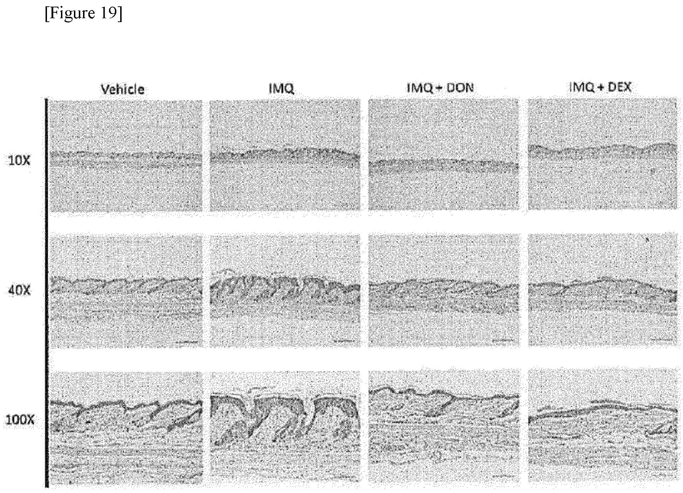

The image of the mouse treated with IMQ or IMQ+therapeutic material is shown in FIG. 18, and the histological analysis result for the skin cross-section of a mouse to observe the morphological change in the skin and the change in influx of immune cells is shown in FIG. 19. Referring to FIGS. 18 and 19, it was observed that, in the control in which the back of a mouse was treated with only IMQ, skin tissue thickened, blisters were formed, and psoriasis accompanied by an erythema symptom was induced, but in the group treated with IMQ and DON, a skin surface at a similar level to normal skin was observed.

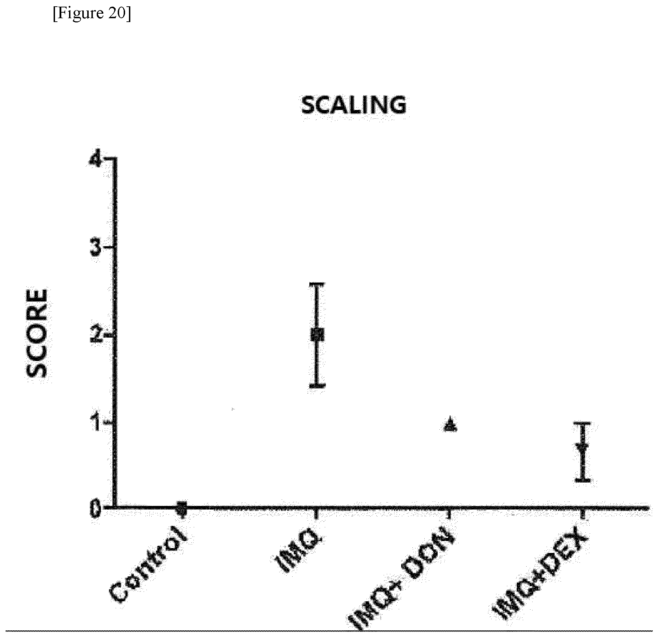

The severity of scaling (FIG. 20) visually evaluated at a lesion site was classified and evaluated by rating, and the severity of the symptom was rated on a 5-point scale (0 = none; 1 = slight; 2 = moderate; 3 = marked; and 4 = very marked), and plotted on a graph. Referring to FIG. 19, it was confirmed that the severity of scaling in the IMQ-treated positive control was significantly increased compared to the negative control, but the severity of scaling in the DON-treated experimental group was significantly decreased compared to the positive control.

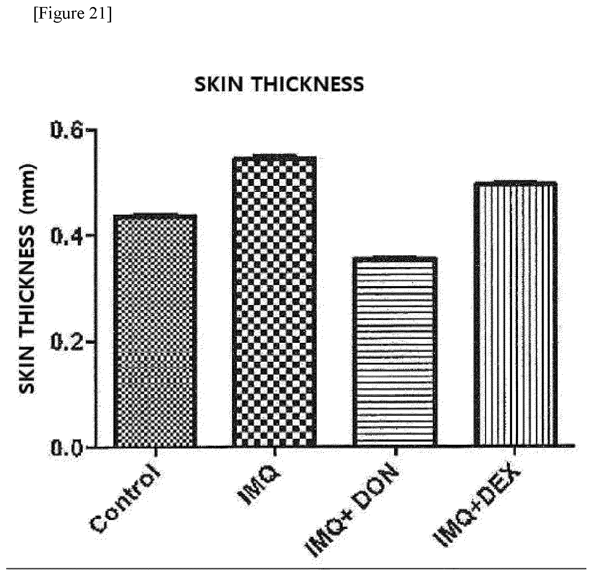

In addition, after the experiment, the skin thickness at the lesion was measured and shown in FIG. 21. As a result of the measurement, in the DON-treated group, the skin thickness was significantly reduced compared to the negative control, confirming that the psoriasis symptom was relieved.

### 7. Confirmation of inhibitory effect of DON on NFκB

This example was intended to indirectly confirm the inhibition of chemokine secretion and histone acetylation by confirming the activity of NFκB in skin cells with a dermatitis lesion, and confirming whether the accelerated NFκB activity was inhibited.

NIH3T3/NFκB-luc cells, which is a cell line capable of confirming the activity of an NFκB transcription factor through luciferase activity were treated with 25 ng/ml of TNF-α to activate an NFκB signal, and as a positive control, 5 µM of a TNF-α inhibitor, Bay11-7082 (Bay), causing an NF-κB signaling inhibitory action was used. NIH3T3/NFκB-luc cells were dispensed at 50x10⁴ cells/well in a 96-well plate, and stabilized in an incubator for 24 hours. To confirm the DON efficacy on NFκB, TNF-α and 100 µM DON were co-treated for 8 hours and then lysed, thereby obtaining a cell enzyme solution, and then the cell enzyme solution was reacted with luciferin, and luciferase activity was measured using a luminometer (FIGS. 3 and 4). The difference in the number of cells between treatment groups was corrected with the resulting values of luciferase activity using protein quantification.

Referring to FIGS. 3 and 4, it was observed that the increase in luciferase activity in a TNF-α-treated group is increased in keratinocytes and fibroblasts, and compared to the TNF-α only-treated group, in the positive control Bay 11-7082 and the TNF-α-co-treated group, it was confirmed that luciferase activity was decreased. Compared to the TNF-α-treated group, in the DON and TNF-α-co-treated group, it was confirmed that luciferase activity was reduced (***P<0.05). From this, it was confirmed that the activation of the NFκB signal induced by TNF-α treatment was inhibited by DON treatment.

## Claims

1. A pharmaceutical composition for use in treating or preventing an inflammatory skin disease, comprising 6-diazo-5-oxo-L-norleucine or a pharmaceutically acceptable salt thereof,
wherein the inflammatory skin disease is caused by chemokine secretion from keratinocytes and fibroblasts.

2. The composition for use of claim 1, wherein the inflammatory skin disease is caused by the hyperproliferation of keratinocytes.

3. The composition for use of claim 1, wherein the inflammatory skin disease is accompanied by pruritus caused by mast cells.

4. The composition for use of claim 1, wherein the inflammatory skin disease is caused by the infiltration of immune cells.

5. The composition for use of claim 1, wherein the inflammatory skin disease is atopic dermatitis.

6. The composition for use of claim 1, wherein the inflammatory skin disease is psoriasis.

7. The composition for use of claim 1, which is orally administered, injected or topically administered to a subject.

8. The composition for use of claim 7, which is administered by the combination of subcutaneous injection and topical administration.

9. The composition for use of claim 7, which is administered by the combination of intradermal injection and topical administration.

10. The composition for use of claim 7, which is injected into a subject at a dose of 0.1 to 5 mg/kg.

11. The composition for use of claim 10, which is administered into a subject twice a week at a dose of 0.1 to 5 mg/kg.

12. The composition for use of claim 7, which is topically administered to a subject at a dose of 0.1 to 5 mg/kg.

13. The composition for use of claim 12, which is administered to a subject at a daily dose of 0.1 to 5 mg/kg.

## Patentansprüche

1. Pharmazeutische Zusammensetzung zur Anwendung bei der Behandlung oder Vorbeugung einer entzündlichen Hautkrankheit, umfassend 6-Diazo-5-oxo-L-norleucin oder ein pharmazeutisch unbedenkliches Salz davon,
wobei die entzündliche Hautkrankheit durch Chemokinsekretion aus Keratinozyten und Fibroblasten verursacht wird.

2. Zusammensetzung zur Anwendung nach Anspruch 1, wobei die entzündliche Hautkrankheit durch die Hyperproliferation von Keratinozyten verursacht wird.

3. Zusammensetzung zur Anwendung nach Anspruch 1, wobei die entzündliche Hautkrankheit von Pruritus begleitet wird, der durch Mastzellen verursacht wird.

4. Zusammensetzung zur Anwendung nach Anspruch 1, wobei die entzündliche Hautkrankheit durch die Infiltration von Immunzellen verursacht wird.

5. Zusammensetzung zur Anwendung nach Anspruch 1, wobei die entzündliche Hautkrankheit atopische Dermatitis ist.

6. Zusammensetzung zur Anwendung nach Anspruch 1, wobei die entzündliche Hautkrankheit Psoriasis ist.

7. Zusammensetzung zur Anwendung nach Anspruch 1, die einem Probanden oral verabreicht, injiziert oder topisch verabreicht wird.

8. Zusammensetzung zur Anwendung nach Anspruch 7, die durch die Kombination von subkutaner Injektion und topischer Verabreichung verabreicht wird.

9. Zusammensetzung zur Anwendung nach Anspruch 7, die durch die Kombination von intradermaler Injektion und topischer Verabreichung verabreicht wird.

10. Zusammensetzung zur Anwendung nach Anspruch 7, die einem Probanden in einer Dosis von 0,1 bis 5 mg/kg injiziert wird.

11. Zusammensetzung zur Anwendung nach Anspruch 10, die einem Probanden zweimal pro Woche in einer Dosis von 0,1 bis 5 mg/kg verabreicht wird.

12. Zusammensetzung zur Anwendung nach Anspruch 7, die einem Probanden in einer Dosis von 0,1 bis 5 mg/kg topisch verabreicht wird.

13. Zusammensetzung zur Anwendung nach Anspruch 12, die einem Probanden in einer täglichen Dosis von 0,1 bis 5 mg/kg verabreicht wird.

## Revendications

1. Composition pharmaceutique pour une utilisation dans le traitement ou la prévention d'une maladie cutanée inflammatoire, comprenant de la 6-diazo-5-oxo-L-norleucine ou un sel pharmaceutiquement acceptable de celle-ci,
dans laquelle la maladie cutanée inflammatoire est causée par la sécrétion de chimiokines à partir de kératinocytes et de fibroblastes.

2. Composition pour une utilisation selon la revendication 1, dans laquelle la maladie cutanée inflammatoire est causée par l'hyperprolifération de kératinocytes.

3. Composition pour une utilisation selon la revendication 1, dans laquelle la maladie cutanée inflammatoire est accompagnée de prurit causé par les mastocytes.

4. Composition pour une utilisation selon la revendication 1, dans laquelle la maladie cutanée inflammatoire est causée par l'infiltration de cellules immunitaires.

5. Composition pour une utilisation selon la revendication 1, dans laquelle la maladie cutanée inflammatoire est une dermatite atopique.

6. Composition pour une utilisation selon la revendication 1, dans laquelle la maladie cutanée inflammatoire est le psoriasis.

7. Composition pour une utilisation selon la revendication 1, qui est administrée par voie orale, injectée, ou administrée par voie topique à un sujet.

8. Composition pour une utilisation selon la revendication 7, qui est administrée par la combinaison d'une injection sous-cutanée et d'une administration topique.

9. Composition pour une utilisation selon la revendication 7, qui est administrée par la combinaison d'une injection intradermique et d'une administration topique.

10. Composition pour une utilisation selon la revendication 7, qui est injectée à un sujet à une dose de 0,1 à 5 mg/kg.

11. Composition pour une utilisation selon la revendication 10, qui est administrée à un sujet deux fois par semaine à une dose de 0,1 à 5 mg/kg.

12. Composition pour une utilisation selon la revendication 7, qui est administrée par voie topique à un sujet à une dose de 0,1 à 5 mg/kg.

13. Composition pour une utilisation selon la revendication 12, qui est administrée à un sujet à une dose quotidienne de 0,1 à 5 mg/kg.
